# EUROPEAN PATENT SPECIFICATION

(11) **EP 2 779 980 B1**
(45) Date of publication and mention of the grant of the patent: **17.08.2016**
(21) Application number: 12813466.5
(22) Date of filing: 14.11.2012
(51) Int. Cl.: A61H 35/04, A61M 11/06

(54) **NASAL IRRIGATION DEVICE**
NASENDUSCHVORRICHTUNG
DISPOSITIF D'IRRIGATION NASALE

(30) Priority: 14.11.2011 IT BS20110155
(43) Date of publication of application: 24.09.2014
(73) Proprietor: MED 2000 S.r.l., 25080 Padenghe Sul Garda, Brescia (IT); Fraccaroli, Davide, 25015 Desenzano del Garda, Brescia (IT)
(72) Inventor: FRACCAROLI, Davide, 25010 Desenzano del Garda, Brescia (IT)
(74) Representative: Chimini, Francesco
(86) International application number: PCT/IB2012/056407
(87) International publication number: WO 2013/072856

(56) References cited:
- EP-A1- 0 652 021
- WO-A1-2009/128109

## Description

The present invention relates to a nasal irrigation device.

In particular, nasal irrigation devices are suitable for being sprayed in the nostrils so as to spray a medical substance therein. In the continuation of the description, medical substance shall be understood to also comprise mere physiological water or other liquid which, when sprayed, helps to dissolve the mucus present in the upper airways.

The devices of the prior art comprise a nozzle, operatively connected to a source of compressed air, and a nozzle-cap, commonly known as a "pisper", making it possible to subject the medical substance to be used to the action of the compressed air so as to be able to spray and dispense it in the nostrils.

The solutions of the prior art present several drawbacks.

For example, said devices comprise a plurality of components for identifying separate chambers so as to be able to easily and separately collect the medical substance to be sprayed and the washing fluid used earlier and sent inside the nostrils. Document EP-A1-0652021 discloses a nasal irrigation device comprising all the technical features set out in the preamble of claim 1.

Such washing fluid must in fact be expelled from the nose and collected separately from the medical substance so as to permit a correct and effective nasal rinse.

The devices of the prior art therefore comprise a plurality of components which make them expensive to make and complicated to use, especially in the dismantling and rinsing operations after use.

The purpose of the present invention is to make a nasal irrigation device which overcomes the drawbacks mentioned in relation to the prior art.

Such drawbacks and limitations are resolved by a device according to claim 1.

Other embodiments of the device according to the invention are described in the subsequent claims.

Further features and advantages of the present invention will be more clearly comprehensible from the description given below of its preferred and non-limiting embodiments, wherein:

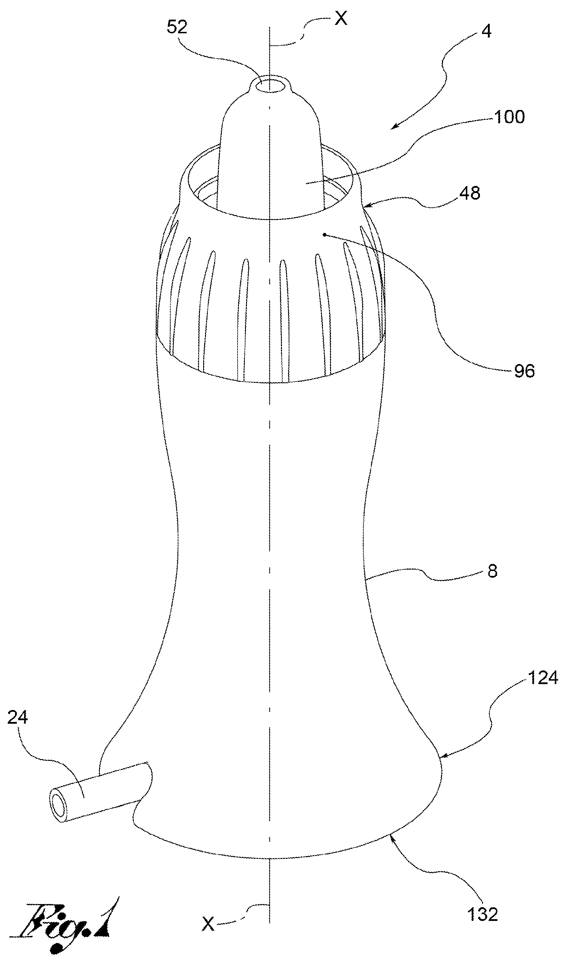

figure 1 shows a perspective view of a nasal irrigation device according to one embodiment of the present invention, in an assembled configuration;

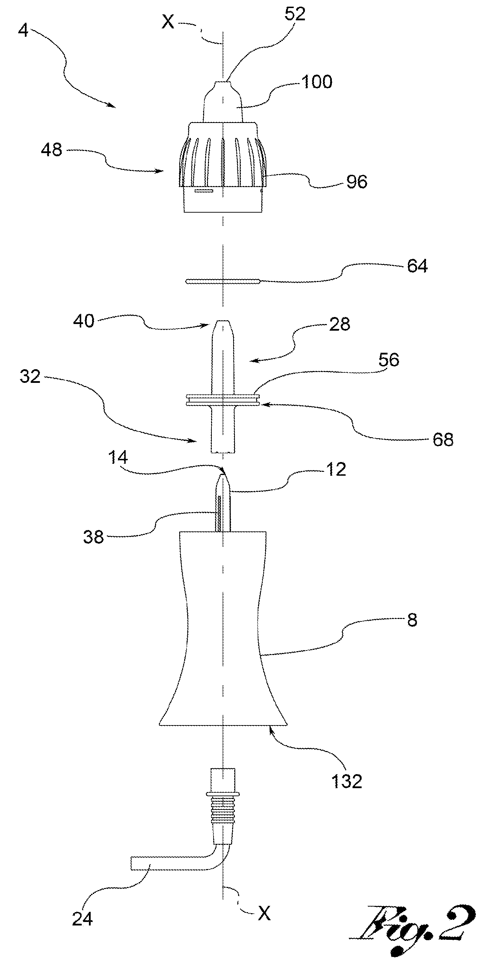

figure 2 shows a view in separate parts, o.f the device in figure 1;

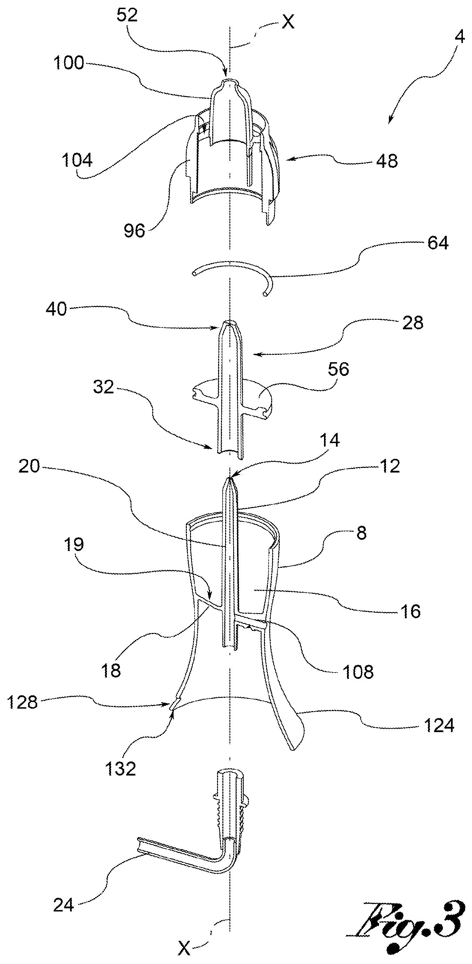

figure 3 shows a cross-section view in separate parts, of the device in figure 1;

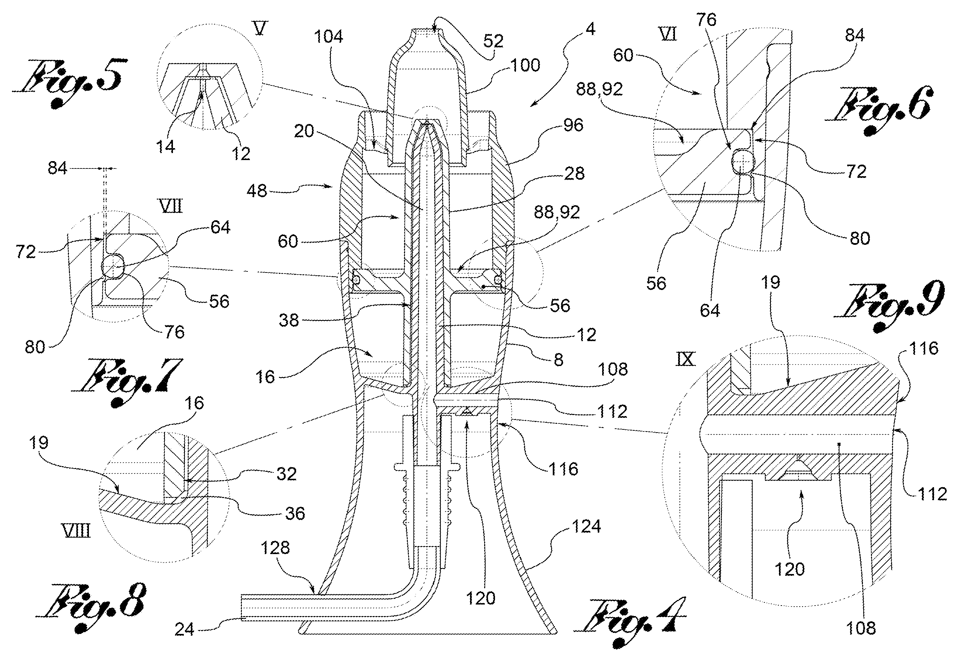

figure 4 shows a further cross-section view, in an assembled configuration, of the device in figure 1;
figures 5, 6, 7, 8, 9 show views of enlarged details V, VI, VII, VIII, IX in figure 4, respectively;
figure 10 shows a perspective view of a nasal irrigation device according to an embodiment variation of the present invention, in an assembled configuration;
figure 11 is an exploded perspective view of the device in figure 10;
figure 12 shows a cross-section view of the device in figure 10 in an assembled configuration; and
figure 13 is a transversal cross-section of the device in figure 10, along the line A-A in figure 12.

The elements or parts of elements common to the embodiments described below will be indicated using the same reference numerals.

With reference to the aforementioned figures, reference numeral 4 globally denotes a nasal irrigation device suitable for being fluidically connected to a compressed air source (not shown), such as for instance the compressor of a medical device for aerosol therapy.

The nasal irrigation device 4 comprises a base body 8 fitted with a nozzle 12 suitable for being fluidically connected to a compressed air source, the nozzle having a dispensing hole 14 of said compressed air.

The base body 8 defines a first chamber 16 for receiving a medical substance to be dispensed through said nozzle 12; said medical substance is received in a liquid phase.

The base body 8 comprises, for example, a lower wall 18 which defines the bottom 19 of said first chamber for the collection of the medical substance.

The nozzle 12 encloses a supply channel 20 of compressed air directed along a main extension X-X and fluidically connected to a compressed air source, for example by means of a connection to a supply tube 24 of compressed air.

Preferably, the nozzle 12 crosses said lower wall 18 and the connection between the nozzle 12 and the supply tube is made under the lower wall 18.

The base body 8 preferably has an hourglass shape suitable for improving the ergonomics of the device 4, facilitating manual gripping thereof by a user.

The device 4 further comprises a nozzle-cap 28 suitable for being fitted at least partially around the nozzle 12 to act in conjunction with it for the dispensing of the medical substance.

In particular, the nozzle-cap 28 in an assembled configuration rests at one of its lower ends 32 on said bottom 19; the lower end 32 comprises at least one slit 36 to permit the uptake of the medical substance collected on the bottom 19.

In a configuration of the nozzle-cap 28 assembled on the nozzle 12, channels 38 are identified between the nozzle-cap 28 and the nozzle 12 to permit the passage of the medical substance from the lower end 32 of the nozzle-cap 28, at which they take up the medical substance from the first chamber 16, to an upper end 40 directly facing the dispensing hole 14 of the nozzle 12.

The upper end 40 of the nozzle-cap 28 is open to permit the passage of the fluid.

For example, said channels 38 may be obtained by making grooves and/or ribs on the side wall of the nozzle 12 or on the inner side wall of the nozzle-cap 28.

The device 4 further comprises a cover 48, fitted on the base body 8, around the nozzle-cap 28, and provided with an exit hole 52 of the medical substance.

The nozzle-cap 28 comprises a separation septum 56 which, acting in conjunction with the cover 48, identifies a second chamber 60, fluidically separate from said first chamber 16, so as to permit the collection of the medical substance used earlier for washing and any mucus dissolved therein. In other words, the separation between the chambers 16, 60 makes it possible to collect the washing fluid, after its use, without it mixing with the medical substance to be sprayed.

According to one embodiment, the separation septum 56 is a disc, positioned coaxially to the nozzle 12, the disc being fitted with sealing means 64 between a lateral rim 68 of the disc and an inner lateral wall 72 of the cover 48. Preferably, said lateral rim 68 of the disc comprises a recess 76 which acts as a seat for sealing means 64, such as, for instance, an O-ring.

The lateral rim 68 of the disc and the inner lateral wall 72 of the cover 48 are shaped so as to form a shaped coupling, to detachably join the separation septum 56 and the cover 48.

For example, the inner lateral wall 72 of the cover 48 comprises a bracket 80 which acts in conjunction with the sealing means 64 of the disc so as to join the cover 48 and the nozzle-cap 28 to each other.

In particular, in an assembled configuration of the cover 48 and the nozzle-cap 28, the bracket 80 interfaces with the sealing means 64 so as to form an undercut with them suitable for countering the mechanical separation of the cover 49 and the nozzle-cap 28. The undercut made by the bracket 80 interferes with the sealing means 64, which are flexible, but not with the separation septum 56 which rather presents a radial clearance 84 with the inner side wall 72.

This way it is possible to couple together and uncouple the cover 48 and the nozzle-cap 28 thanks to the elastic deformation of the sealing means 64 at the interface with the bracket 80

The separation septum 56 comprises a hollow seat 92 on the upper side 88 facing the associable cover 48 suitable for favouring the collection of the medical substance used earlier for washing.

The cover 48 comprises a cover body 96 and a cap 100 having said exit hole 52.

The cap 100 is positioned coaxially to the nozzle-cap 28 so as to channel the outgoing fluid from the dispensing hole 14 of the nozzle 12 towards said exit hole 52. Preferably, between the cap 100 and the cover body 96 apertures are present 104 which open onto the second chamber 60, so as to enable drainage of the used medical substance into the second chamber 60.

Preferably, said apertures are positioned coaxially to the cap 100.

According to one embodiment, the base body 8 comprises an activation channel 108 which fluidically intercepts the supply channel 20 and which ends externally in an activation hole 112 made on an outer side wall 116 of the base body 8 so as to be easily occluded by the user by pressure of the fingers.

Preferably, the activation channel 108 is made inside the lower wall 18 which defines the bottom 19 of the first chamber 16 for the collection of the medical substance.

The opening, that is to say the through cross-section, of the activation channel 108 and especially of the activation hole 112 is greater than the opening of the dispensing hole 14 of the nozzle 12. This way, while the activation hole 112 remains open, the compressed air coming from the lower end 32 of the nozzle 12 tends to flow out through the activation channel 108 and relative activation hole 112 without coming out of the dispensing hole 14.

Preferably, the activation channel 108 comprises a vent hole 120, positioned between the supply channel 20 and the activation hole 108, wherein the vent hole 120 is calibrated so as to prevent overpressures inside the nozzle 12.

Preferably, the vent hole 120 faces towards a support base 124 of the base body 8 and is enclosed inside the base body 8 so as to prevent the possible projection of fluid against the user, in the event of fluid coming out from the vent hole 120.

According to one embodiment, the support base 124 of the base body 8 comprises a slot 128 for the passage of a supply tube 24 of compressed air, said slot 128 coming out on the lower rim 132 of the support base 124 so as to permit a stable support of the device 4 even in the configuration of the supply tube being fitted.

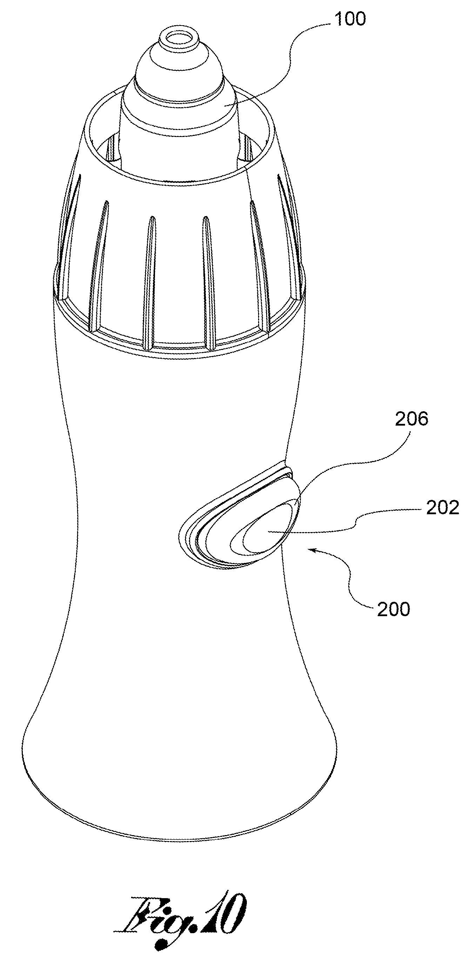

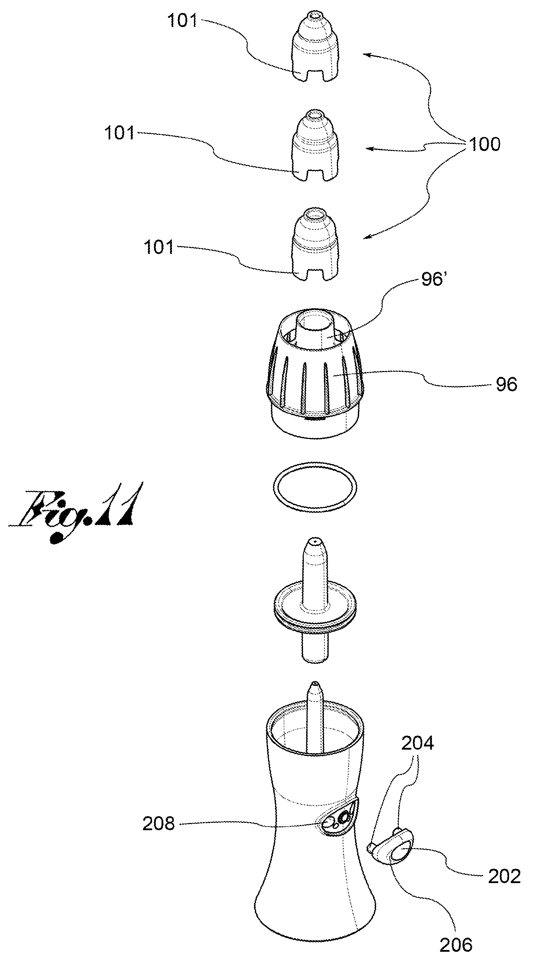

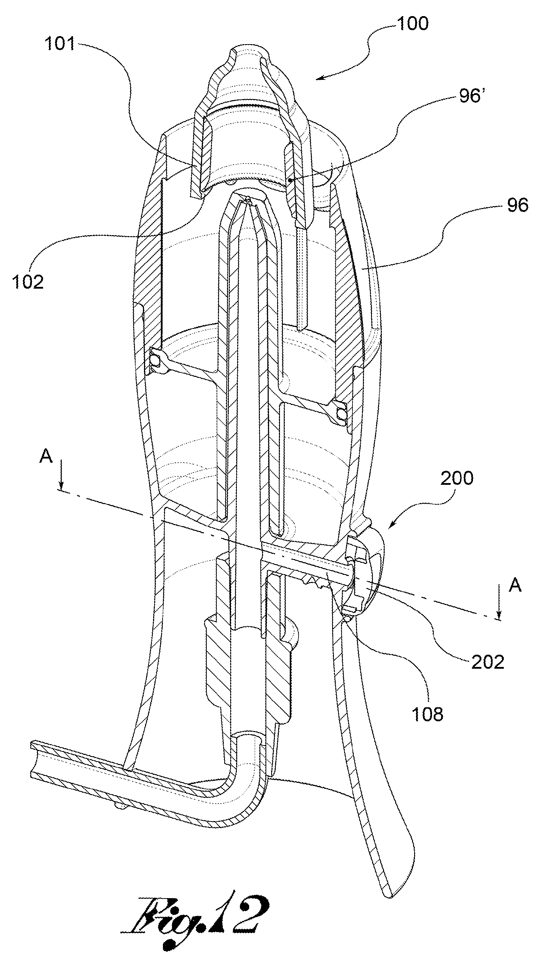

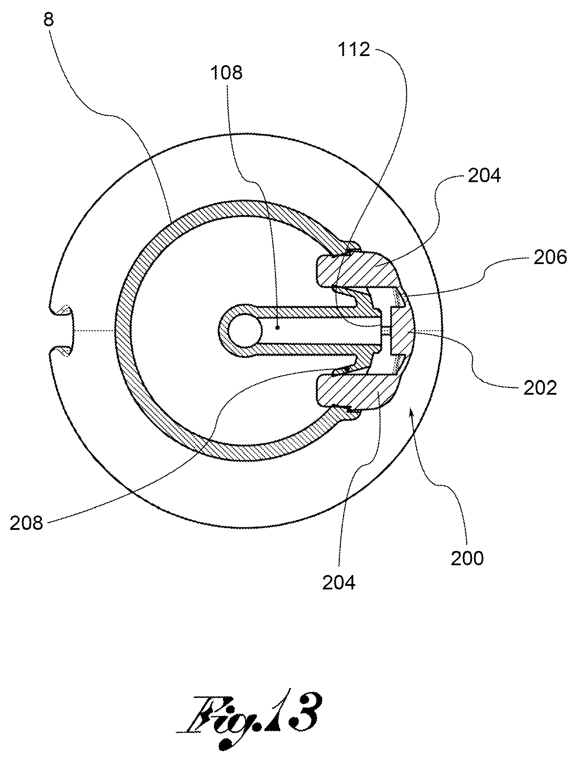

In one embodiment variation shown in figures 10-13, an activation button 200 is associated with the activation hole 11 suitable for occluding said hole 112.

In particular, said activation button 200 comprises a central actuation portion 202, facing the activation hole 112, a pair of lateral arms 204, and a thin membrane 206 which connects said central actuation portion 202 to the lateral arms 204. The latter are inserted with interference in respective conical seats 208 made in the base body 8.

The activation button 200 is thus permanently attached to the base body 8 thanks to the lateral arms 204 inserted without the possibility of extraction in the respective conical seats 208. The thin membrane 206 allows the user to press the central actuation portion 202 against the activation hole 112 and to return to a rearward open position of said hole upon ceasing of pressure exerted by the user.

Advantageously, the activation button 200 is made in a single piece, does not require separate attachment means to the base body 8 and does not need separate elastic means for its return to an inactive position.

In the embodiment variation shown in figures 10-13, moreover, the cap 100 with the exit hole 52 is separate from the cover body 96, so as to permit the use of different, interchangeable, caps 100, differing for example in the dimensions of the end part suitable for being inserted in the user's nostril.

In particular, the cover body 96 is fitted with a truncated-cone or cylindrical collar 96', for example made in one piece with said cover body 96, which extends coaxially round the upper end 40 of the nozzle-cap 28 and which acts as a means of coupling to a cap 100.

Each cap 100 is suitable for being fitted on said collar 96' and is provided at the bottom with coupling sectors 101 fitted with coupling teeth 102 which snap engage into the undercut defined by the lower rim of the collar 96'.

The functioning and assembly of a nasal irrigation device according to the invention will now be described.

In particular, after connecting the device 4 to a compressed air dispenser, such as for example a device for aerosol therapy, the user may activate the dispensing of compressed air.

Said air flows from the supply tube of compressed air into the nozzle 12: if the activation hole 112 is covered by the user's finger or by the activation button 200, the compressed air is dispensed through the dispensing hole 14 of the nozzle.

Coming out from said activation hole 14, the air creates a depression which aspirates the medical substance contained in the first chamber: said medical substance can in fact move up from the first chamber 16 through the slits and channels as far as the dispensing hole 14 where it mixes with the compressed air.

The mixture thus obtained can then pass through the open upper end 40 of the nozzle-cap 28, the cover 48 and then be dispensed in the nostrils through the exit hole 52.

If the user removes his finger from the activation hole 112 or releases the activation button 200, the air comes out through said activation hole 112 which has a larger opening than the dispensing hole 14 of the nozzle 12 and, consequently, offers less resistance to the passage of air than the dispensing hole 14 of the nozzle 12.

This way, whenever the user wishes to suspend even solely for an instant the nasal irrigation treatment, he can simply remove his finger from the activation hole 112 so as to expel the compressed air laterally in relation to the base body 8 without dispensing medical substance which would otherwise be wasted. This operation can be performed instantly and in an extremely easy and intuitive manner without having to turn off the dispensing device of compressed air every time.

In fact, with the devices of the prior art the user would have to turn off the dispenser device of compressed air each time he needs to distance the device from his nostrils to interrupt the treatment, to prevent wastage of medical substance.

In addition, the configuration of the lower rim 132 of the base body 8, provided with a hole or slot 128 for the passage of the supply tube 24, makes use of the nasal irrigation device 4 even easier.

In fact, despite the supply tube 24, the device 4 may still be stably rested for example on a shelf: this way it is even easier to suspend and resume the nasal irrigation treatment. In fact, every time the user wishes to suspend treatment, he can merely take his finger off the activation hole 112, to prevent the dispersion of medical substance in the air, and rest the device 4 on a shelf in a stable manner given that the supply tube 24 does not interfere with the lower rim 132 of the base body 8.

This operation may be easily repeated providing at all times a valid support for the device and preventing any wastage of medical substance without having to turn off the dispenser device of compressed air each time.

During the treatment, the washing fluid continually flows back towards the cap 100 by gravity and, passing through the apertures 104, falls into the second chamber 60 so as to remain separate at all times from the first chamber 16 containing the medical substance yet to be dispensed.

At the end of treatment, the cover 48 can be extracted taking with it the attached nozzle-cap 28: the cover 48 and nozzle-cap 28 assembly encloses the second chamber 60 inside which the used washing fluid is collected. By dismantling the cover 48 the second chamber can be easily cleaned. In addition, following removal of the cover 48 and nozzle-cap 28 assembly, the first chamber can be easily accessed to clean it or if needed, fill it with new medical substance.

As may be appreciated from the description, the nasal irrigation device according to the invention makes it possible to overcome the drawbacks of the prior art presented.

In particular, the device according to the present invention is particularly convenient and easy to use.

In fact the device comprises a limited number of components which are easy to assemble and separate so as to permit easy filling of the device with the medical substance but also easy emptying thereof after use.

In particular the step of cleaning the device after use is particularly facilitated. In fact, after the washing, the medical substance has already flowed back into the second chamber: the cap can then be detached taking with it the nozzle-cap with which it delimits the second chamber.

The nozzle-cap can then be separated from the cap so as to permit easy cleaning of the second chamber. At the same time the nozzle and first chamber can also be cleaned, easily accessible following the joint removal of the cap and nozzle-cap.

Moreover the device proves very easy to use during nasal irrigation.

In fact, as seen, the presence of the activation hole makes it possible to activate or prevent the dispensing of compressed air, and therefore of medical substance, from the dispensing hole, without having to turn off the dispensing device of compressed air.

In addition, the configuration of the lower wall of the base body, provided with a hole or slot 128 for the passage of the supply tube 24, makes use of the nasal irrigation device 4 even easier.

A person skilled in the art may make numerous modifications and variations to the nasal irrigation devices described above so as to satisfy contingent and specific requirements, all contained within the scope of the invention as defined by the appended claims.

## Claims

1. Nasal irrigation device (4) comprising
- a base body (8) fitted with a nozzle (12) suitable for being fluidically connected to a compressed air source, the nozzle (12) having a dispensing hole (14) of said compressed air,
- the base body (8) defining a first chamber (16) for receiving a medical substance to be dispensed through said nozzle (12),
- a nozzle-cap (28) suitable for being fitted at least partially around the nozzle (12) to act in conjunction with it for the dispensing of the medical substance, wherein channels (38) are identified between the nozzle-cap (28) and the nozzle (12) to permit the passage of the medical substance from a lower end (32) of the nozzle-cap (28) at which they take up the medical substance from the first chamber (16) to an upper end (40) directly facing the dispensing hole (14) of the nozzle (12),
- a cover (48) fitted on the base body (8), around the nozzle-cap (28), and provided with an exit hole (52) of the medical substance,
**characterised in that**
- the nozzle-cap (28) comprises a separation septum (56) which, acting in conjunction with the cover (48), identifies a second chamber (60), fluidically separate from said first chamber (16) so as to permit the collection of the medical substance used earlier for washing.

2. Nasal irrigation device (4) according to claim 1, wherein the separation septum (56) is a disc, positioned coaxially to the nozzle (12), the disc being fitted with sealing means (64) between the lateral rim (68) of the disc and an inner lateral wall (72) of the cover (48).

3. Nasal irrigation device (4) according to claim 2, wherein the lateral rim (68) of the disc and the inner lateral wall (72) of the cover (48) are shaped according to a shaped coupling, so as to detachably join the separation septum (56) and the cover (48).

4. Nasal irrigation device (4) according to claim 2 or 3, wherein the inner lateral wall (72) of the cover (48) comprises a bracket (80) which acts in conjunction with the sealing means (64) of the disc so as to join the cover (48) and the nozzle-cap (28) to each other.

5. Nasal irrigation device (4) according to any of the previous claims, wherein the separation septum (56), comprises a hollow seat (92) on the upper side (88) facing the associable cover (48) suitable for favouring the collection of the medical substance used earlier for washing.

6. Nasal irrigation device (4) according to any of the previous claims, wherein the base body (8) comprises a lower part (18) which defines a bottom (19) of said first chamber (16) for the collection of the medical substance, and in which the nozzle-cap (28) in an assembled configuration rests on one of its lower ends (32) on said bottom (19), said lower end (32) comprising at least one slit (36) to permit the uptake of the medical substance collected on the bottom (19).

7. Nasal irrigation device (4) according to any of the previous claims, wherein the cover (48) comprises a cover body (96) and a cap (100) having an exit hole (52), the cap (100) channelling the outgoing fluid from the dispensing hole (14) of the nozzle (12) towards said exit hole (52), and wherein between the cap (100) and the cover body (96) apertures are present (104) which open onto the second chamber (60), so as to enable drainage of the used medical substance into the second chamber (86).

8. Nasal irrigation device (4) according to any of the previous claims, wherein the nozzle (12) encloses a supply channel (20) of compressed air directed along a main extension (X-X) and fluidically connected to a compressed air source, and wherein the base body (8) comprises an activation channel (108) which fluidically intercepts the supply channel (20) and which ends externally in an activation hole (112) made on an outer lateral wall (116) of the base body (8) so as to be easily occluded by the user by pressure of the fingers.

9. Nasal irrigation device (4) according to claim 8, wherein said activation channel (108) is made inside a lower wall (18) of the base body (8) which defines the bottom (19) of said first chamber (16) for the collection of the medical substance.

10. Nasal irrigation device (4) according to claim 8 or 9, wherein said activation channel (108) comprises a vent hole (120), positioned between the supply channel (20) and the activation hole (112), the vent hole (120) being calibrated so as to prevent over pressure inside the nozzle (12).

11. Nasal irrigation device (4) according to claim 10, wherein said vent hole (120) faces towards a support base (124) of the base body (8) and is enclosed inside the base body (8) so as to prevent the possible projection of the fluid against the user.

12. Nasal irrigation device (4) according to any of the previous claims, wherein a support base (124) of the base body (8) comprises a slot (128) for the passage of a supply tube (24) of compressed air, said slot (128) coming out on a lower rim (132) of the support base (124) so as to permit stable support of the device (4) even in the configuration of the supply tube being fitted.

13. Nasal irrigation device (4) according to any of the claims 8-12, wherein an activation button (200) is associated to the activation hole (112) suitable for occluding said activation hole (112), said activation button (200) comprising a central actuation portion (202), facing the activation hole (112), a pair of lateral arms (204) inserted with interference in respective conical seats (208) made in the base body (8), and a thin membrane (206) which connects said central actuation portion (202) to the lateral arms (204) so as to permit the excursion of the central actuation portion (202)between a rearward inactive position and an advanced occlusion position of the activation hole (112).

14. Nasal irrigation device (4) according to any of the claims 7-13, wherein the cap (100) is connected in a detachable manner to the body cover (96), said cap (100) and said body cover (96) being provided with reciprocal snap coupling means (96', 101,102).

## Patentansprüche

1. Nasenspülvorrichtung (4), umfassend:
- einen Grundkörper (8), welcher mit einer Düse (12) ausgestattet ist, welche geeignet ist, fluidisch mit einer Quelle für komprimierte Luft verbunden zu werden, wobei die Düse (12) ein Ausgabeloch (14) für diese komprimierte Luft aufweist,
- wobei der Grundkörper (8) eine erste Kammer (16) zum Aufnehmen einer medizinischen Substanz definiert, welche durch die Düse (12) abzugeben ist,
- eine Düsenkappe (28), welche geeignet ist, wenigstens teilweise um die Düse (12) herum gepasst zu werden, um in Verbindung mit ihr zum Abgeben der medizinischen Substanz zu wirken, wobei Kanäle (38) zwischen der Düsenkappe (28) und der Düse (12) ausgebildet sind, um den Durchgang der medizinischen Substanz von einem unteren Ende (32) der Düsenkappe (28), an welchem sie die medizinische Substanz aus der ersten Kammer (16) aufnehmen, zu einem oberen Ende (40), welches direkt zu dem Ausgabeloch (14) der Düse (12) weist, zu erlauben,
- eine Abdeckung (48), welche auf den Grundkörper (8) um die Düsenkappe (28) herum gepasst und mit einem Ausgangsloch (52) für die medizinische Substanz bereitgestellt ist,
**dadurch gekennzeichnet, dass**
die Düsenkappe (28) eine Trennwand (56) umfasst, welche in Verbindung mit der Abdeckung (48) wirkend eine zweite Kammer (60) ausbildet, welche von der ersten Kammer (16) fluidisch getrennt ist, um so das Sammeln der medizinischen Substanz zu erlauben, die zuvor zum Waschen verwendet worden ist.

2. Nasenspülvorrichtung (4) nach Anspruch 1, wobei die Trennwand (56) eine Scheibe ist, welche koaxial mit der Düse (12) positioniert ist, wobei die Scheibe mit Dichtungsmitteln (64) zwischen dem lateralen Rand (68) der Scheibe und einer inneren lateralen Wand (72) der Abdeckung (48) ausgestattet ist.

3. Nasenspülvorrichtung (4) nach Anspruch 2, wobei der laterale Rand (68) der Scheibe und die innere laterale Wand (72) der Abdeckung (48) gemäß einer Formkopplung geformt sind, um so lösbar die Trennwand (56) und die Abdeckung (48) zu verbinden.

4. Nasenspülvorrichtung (4) nach Anspruch 2 oder 3, wobei die innere laterale Wand (72) der Abdeckung (48) eine Halterung (80) umfasst, welche in Verbindung mit den Dichtungsmitteln (64) der Scheibe wirkt, um die Abdeckung (48) und die Düsenkappe (28) miteinander zu verbinden.

5. Nasenspülvorrichtung (4) nach einem der vorhergehenden Ansprüche, wobei die Trennwand (56) einen hohlen Sitz (92) an der oberen Seite (88) zu der zuordenbaren Abdeckung (48) weisend umfasst, welcher geeignet ist, das Sammeln der zuvor zum Waschen verwendeten medizinischen Substanz zu unterstützen.

6. Nasenspülvorrichtung (4) nach einem der vorhergehenden Ansprüche, wobei der Grundkörper (8) einen unteren Teil (18) umfasst, welcher einen Boden (19) der ersten Kammer (16) zum Sammeln der medizinischen Substanz definiert, und wobei die Düsenkappe (28) in einer montierten Konfiguration auf einem ihrer unteren Enden (32) an dem Boden (19) ruht, wobei das untere Ende (32) wenigstens einen Schlitz (36) umfasst, um die Aufnahme der an dem Boden (19) gesammelten medizinischen Substanz zu erlauben.

7. Nasenspülvorrichtung (4) nach einem der vorhergehenden Ansprüche, wobei die Abdeckung (48) einen Abdeckungskörper (96) und eine Kappe (100) mit einem Ausgangsloch (52) umfasst, wobei die Kappe (100) das ausgehende Fluid von dem Ausgabeloch (14) der Düse in Richtung des Ausgangslochs (52) kanalisiert, und wobei zwischen der Kappe (100) und dem Abdeckungskörper (96) Öffnungen vorliegen (104), die sich in die zweite Kammer (60) öffnen, um so ein Abführen der verwendeten medizinischen Substanz in die zweite Kammer (86) zu erlauben.

8. Nasenspülvorrichtung (4) nach einem der vorhergehenden Ansprüche, wobei die Düse (12) einen Zufuhrkanal (20) für komprimierte Luft einschließt, welcher entlang einer Haupterstreckung (X-X) gerichtet und fluidisch mit einer Quelle für komprimierte Luft verbunden ist, und wobei der Grundkörper (8) einen Aktivierungskanal (108) umfasst, welcher fluidisch den Zufuhrkanal (20) unterbricht, und welcher extern in einem Aktivierungsloch (112) endet, welches an einer äußeren lateralen Wand (116) des Grundkörpers (8) gebildet ist, um so leicht von dem Benutzer durch Druck der Finger verschlossen zu werden.

9. Nasenspülvorrichtung (4) nach Anspruch 8, wobei der Aktivierungskanal (108) innerhalb einer unteren Wand (18) des Grundkörpers (8) gebildet ist, welche den Boden (19) der ersten Kammer (16) zum Sammeln der medizinischen Substanz definiert.

10. Nasenspülvorrichtung (4) nach Anspruch 8 oder 9, wobei der Aktivierungskanal (108) ein Belüftungsloch (120) umfasst, welches zwischen dem Zufuhrkanal (20) und dem Aktivierungsloch (112) positioniert ist, wobei das Belüftungsloch (120) derart kalibriert ist, dass ein Überdruck innerhalb der Düse (12) verhindert wird.

11. Nasenspülvorrichtung (4) nach Anspruch 10, wobei das Belüftungsloch (120) in Richtung einer Tragebasis (124) des Grundkörpers (8) weist und innerhalb des Grundkörpers (8) eingeschlossen ist, um das mögliche Vorspringen des Fluids gegen den Benutzer zu verhindern.

12. Nasenspülvorrichtung (4) nach einem der vorhergehenden Ansprüche, wobei eine Tragebasis (124) des Grundkörpers (8) eine Ausnehmung (128) für den Durchgang einer Zufuhrröhre (24) für komprimierte Luft umfasst, wobei die Ausnehmung (128) an einem unteren Rand (132) der Tragebasis (124) herauskommt, um so ein stabiles Tragen der Vorrichtung (4) selbst in der Konfiguration zu erlauben, in welcher die Zufuhrröhre eingepasst ist.

13. Nasenspülvorrichtung (4) nach einem der Ansprüche 8-12, wobei ein Aktivierungsknopf (200) dem Aktivierungsloch (112) zugeordnet ist, welcher geeignet ist, das Aktivierungsloch (112) zu verschließen, wobei der Aktivierungsknopf (200) einen zentralen Betätigungsabschnitt (202), welcher zu dem Aktivierungsloch (112) weist, ein Paar von lateralen Armen (204), welche mit Eingriff in jeweilige konische Sitze (208) eingesetzt sind, welche in dem Grundkörper (8) gebildet sind, und eine dünne Membran (206) umfasst, welche den zentralen Betätigungsabschnitt (202) mit den lateralen Armen (204) verbindet, um so die Überführung des zentralen Betätigungsabschnitts (202) zwischen einer hinteren inaktiven Position und einer vorgeschobenen Verschlussposition des Aktivierungslochs (112) zu erlauben.

14. Nasenspülvorrichtung (4) nach einem der Ansprüche 7-13, wobei die Kappe (100) in einer lösbaren Weise mit der Körperabdeckung (96) verbunden ist, wobei die Kappe (100) und die Körperabdeckung (96) mit entgegengesetzten Schnapp-Kopplungsmitteln (96', 101, 102) bereitgestellt sind.

## Revendications

1. Dispositif d'irrigation nasale (4) comprenant
- un corps de base (8) équipé d'une buse (12) appropriée pour être reliée de manière fluidique à une source d'air comprimé, la buse (12) ayant un orifice de distribution (14) dudit air comprimé,
- le corps de base (8) définissant une première chambre (16) pour recevoir une substance médicale à distribuer à travers ladite buse (12),
- un capuchon de buse (28) approprié pour être ajusté au moins partiellement autour de la buse (12) pour agir conjointement avec elle pour la distribution de la substance médicale, dans lequel des canaux (38) sont identifiés entre le capuchon de buse (28) et la buse (12) pour permettre le passage de la substance médicale depuis une extrémité inférieure (32) du capuchon de buse (28), au niveau de laquelle ils font remonter la substance médicale de la première chambre (16) jusqu'à une extrémité supérieure (40) faisant directement face à l'orifice de distribution (14) de la buse (12),
- un couvercle (48) ajusté sur le corps de base (8), autour du capuchon de buse (28), et muni d'un orifice de sortie (52) de la substance médicale,
**caractérisé en ce que**
- le capuchon de buse (28) comprend une cloison de séparation (56) qui, agissant conjointement avec le couvercle (48), définie une seconde chambre (60), séparée de manière fluidique de ladite première chambre (16) de manière à permettre le recueil de la substance médicale utilisée précédemment pour un lavage.

2. Dispositif d'irrigation nasale (4) selon la revendication 1, dans lequel la cloison de séparation (56) est un disque, positionné de manière coaxiale à la buse (12), le disque étant muni d'un moyen d'étanchéité (64) entre le bord latéral (68) du disque et une paroi latérale interne (72) du couvercle (48).

3. Dispositif d'irrigation nasale (4) selon la revendication 2, dans lequel le bord latéral (68) du disque et la paroi latérale interne (72) du couvercle (48) sont formés selon un couplage profilé, de manière à joindre de manière détachable la cloison de séparation (56) et le couvercle (48).

4. Dispositif d'irrigation nasale (4) selon la revendication 2 ou 3, dans lequel la paroi latérale interne (72) du couvercle (48) comprend une console (80) qui agit conjointement avec le moyen d'étanchéité (64) du disque de manière à joindre le couvercle (48) et le capuchon de buse (28) l'un à l'autre.

5. Dispositif d'irrigation nasale (4) selon l'une quelconque des revendications précédentes, dans lequel la cloison de séparation (56) comprend un siège creux (92) sur le côté supérieur (88) faisant face au couvercle (48) associable approprié pour favoriser le recueil de la substance médicale utilisée précédemment pour un lavage.

6. Dispositif d'irrigation nasale (4) selon l'une quelconque des revendications précédentes, dans lequel le corps de base (8) comprend une partie inférieure (18) qui définit un fond (19) de ladite première chambre (16) pour le recueil de la substance médicale, et dans lequel le capuchon de buse (28), dans une configuration assemblée, repose sur l'une de ses extrémités inférieures (32) sur ledit fond (19), ladite extrémité inférieure (32) comprenant au moins une fente (36) pour permettre la remontée de la substance médicale recueillie sur le fond (19).

7. Dispositif d'irrigation nasale (4) selon l'une quelconque des revendications précédentes, dans lequel le couvercle (48) comprend un corps de couvercle (96) et un capuchon (100) ayant un orifice de sortie (52), le capuchon (100) canalisant le fluide sortant de l'orifice de distribution (14) de la buse (12) en direction dudit orifice de sortie (52), et dans lequel sont présentes, entre le capuchon (100) et le corps de couvercle (96), des ouvertures (104) qui débouchent sur la seconde chambre (60), de manière à permettre l'évacuation de la substance médicale usagée dans la seconde chambre (86).

8. Dispositif d'irrigation nasale (4) selon l'une quelconque des revendications précédentes, dans lequel la buse (12) entoure un canal d'alimentation (20) d'air comprimé dirigé le long d'une extension principale (X-X) et relié de manière fluidique à une source d'air comprimé, et dans lequel le corps de base (8) comprend un canal d'activation (108) qui intercepte de manière fluidique le canal d'alimentation (20) et qui se termine à l'extérieur dans un orifice d'activation (112) réalisé sur une paroi latérale extérieure (116) du corps de base (8) de manière à être facilement bouché par l'utilisateur par une pression des doigts.

9. Dispositif d'irrigation nasale (4) selon la revendication 8, dans lequel ledit canal d'activation (108) est réalisé à l'intérieur d'une paroi inférieure (18) du corps de base (8) qui définit le fond (19) de ladite première chambre (16) pour le recueil de la substance médicale.

10. Dispositif d'irrigation nasale (4) selon la revendication 8 ou 9, dans lequel ledit canal d'activation (108) comprend un orifice d'aération (120) positionné entre le canal d'alimentation (20) et l'orifice d'activation (112), l'orifice d'aération (120) étant calibré de manière à empêcher une surpression à l'intérieur de la buse (12).

11. Dispositif d'irrigation nasale (4) selon la revendication 10, dans lequel ledit orifice d'aération (120) est orienté vers une base de support (124) du corps de base (8) et est enfermé à l'intérieur du corps de base (8) de manière à empêcher la projection éventuelle du fluide sur l'utilisateur.

12. Dispositif d'irrigation nasale (4) selon l'une quelconque des revendications précédentes, dans lequel une base de support (124) du corps de base (8) comprend une encoche (128) pour le passage d'un tube d'alimentation (24) d'air comprimé, ladite encoche (128) faisant saillie sur un bord inférieur (132) de la base de support (124) de manière à permettre un soutien stable du dispositif (4) même dans la configuration où le tube d'alimentation est en place.

13. Dispositif d'irrigation nasale (4) selon l'une quelconque des revendications 8 à 12, dans lequel un bouton d'activation (200) est associé à l'orifice d'activation (112) approprié pour boucher ledit orifice d'activation (112), ledit bouton d'activation (200) comprenant une partie d'actionnement centrale (202), faisant face à l'orifice d'activation (112), une paire de bras latéraux (204) insérés avec interférence dans des sièges coniques respectifs (208) réalisés dans le corps de base (8), et une fine membrane (206) qui relie ladite partie d'actionnement centrale (202) aux bras latéraux (204) de manière à permettre la course de la partie d'actionnement centrale (202) entre une position inactive arrière et une position d'occlusion avancée de l'orifice d'activation (112).

14. Dispositif d'irrigation nasale (4) selon l'une quelconque des revendications 7 à 13, dans lequel le capuchon (100) est relié de manière détachable au couvercle du corps (96), ledit capuchon (100) et ledit couvercle du corps (96) étant muni de moyens de couplage à enclenchement réciproque (96', 101,102).
